# EUROPEAN PATENT SPECIFICATION

(11) **EP 3 668 714 B1**
(45) Date of publication and mention of the grant of the patent: **06.10.2021**
(21) Application number: 18762416.8
(22) Date of filing: 02.08.2018
(51) Int. Cl.: B32B 37/02, B32B 37/15, A61F 13/15, B32B 5/02, B32B 7/12, B32B 27/12

(54) **IN-LINE PROCESS OF PRODUCING A POLYMER FILM FOR DISPOSABLE HYGIENE PRODUCTS**
IN-LINE-VERFAHREN ZUR HERSTELLUNG EINES POLYMERFILMS FÜR WEGWERFHYGIENEPRODUKTE
PROCÉDÉ EN LIGNE DE PRODUCTION D'UN FILM POLYMÈRE POUR PRODUITS HYGIÉNIQUES JETABLES

(30) Priority: 16.08.2017 US 201762546380 P
(43) Date of publication of application: 24.06.2020
(73) Proprietor: Illinois Tool Works Inc., Glenview, IL 60025 (US)
(72) Inventor: LESSLEY, Mel Steven, Glenview, IL 60025 (US)
(74) Representative: HGF
(86) International application number: PCT/US2018/044921
(87) International publication number: WO 2019/036200

(56) References cited:
- WO-A1-2004/049989
- US-A1- 2003 065 297
- US-A1- 2007 131 335
- US-B2- 7 507 680

## Description

### BACKGROUND

Disposable hygiene products typically include a core layer including, for example, an absorbent layer or pad, disposed between a top sheet and a back sheet. Disposable hygiene products may further include elastics around leg openings and/or a waist opening, for example, to improve fit on a wearer, and fasteners to hold the product in a desired position during use.

The core layer may be produced along a manufacturing line and may be made from, for example, synthetic or natural fibers with absorbency characteristics. The top sheet and back sheet are formed in a process separate from the production of the core layer and apart from the manufacturing line. For example, the back sheet may for made from a polymer film that may optionally be laminated to a non-woven material layer. The back sheet, and in particular, the film of the back sheet, is a non-permeable layer may be made from, for example, polyethylene or polypropylene. Typically, the polymer film for the back sheet will be fabricated at a supplier manufacturing facility, packaged as a roll of film and transported to the disposable hygiene product manufacturing facility. The roll of polymer film is typically bulky and heavy, and may be difficult and/or expensive to transport and maneuver. The top sheet is typically formed of a non-woven material. The top sheet is a permeable layer and may be made from, for example, polypropylene.

To assemble the disposable hygiene product, the polymer film may be fed from the roll of material, along a machine direction to form a bottom layer, or back sheet, of the product. Subsequently, the core layer, such as the absorbent layer or pad, leg / cuff elastics and a waistband may be applied to the back sheet. The top sheet is fed along a machine direction, applied over the back sheet and the core layer and laminated thereto. The various layers, sheets and/or individual components of the disposable hygiene product may be laminated to one another, or other individual components, with an adhesive, heating or ultrasonic welding. Such lamination may occur in-line with the manufacturing by, for example, positioning adhesive applicators at various locations along the line. The combined laminate may then be cut into individual sections to form corresponding disposable hygiene products.

In some products, the back sheet is a laminate formed by the polymer film and a nonwoven material. In such a back sheet, the polymer film and nonwoven material may be laminated using additional adhesives, heat or ultrasonic welding, for example. Lamination of the nonwoven material and polymer film may occur at a location remote of the disposable hygiene product manufacturing line. The laminated back sheet may then be transported to the disposable hygiene manufacturing facility as described above. In another configuration, the polymer film and nonwoven material may be laminated together in the disposable hygiene product manufacturing line. However, similar to the process above, additional adhesive, heat, or ultrasonic welding steps, or the like, are required to bond the film and the nonwoven material together. This requires additional manufacturing steps, equipment, and time to produce the disposable hygiene product. Additional space may be required at the disposable hygiene product facility as well, to accommodate the roll of material and laminating equipment. Methods of forming a disposable hygiene product according to the background art are known from documents US 2003/065297 A1, WO 2004/049989 A1 and US 7 507 680 B2.

Accordingly, it is desirable to provide an apparatus and process for making a disposable hygiene product in which the back sheet may be simultaneously, or nearly simultaneously, produced, in-line with the manufacture of the disposable hygiene product. In addition, it may be desirable to provide an apparatus and process in which the back sheet includes a polymer film and a nonwoven material, and the polymer film and nonwoven material may be bonded together in-line with the manufacturing process, without requiring additional bonding steps or equipment.

### SUMMARY

According to one aspect, there is provided a method of forming a disposable hygiene product. The method includes providing a manufacturing line having a back sheet station, a core layer station and a top sheet station connected by a conveying system, providing a back sheet at the back sheet station, providing a core layer at the core layer station, applying the core layer on the back sheet, providing a top sheet at the top sheet station and applying the top sheet on the back sheet and core layer. The back sheet station comprises a fluid application device configured to discharge a curtain of material to form a polymer film, wherein the back sheet comprises the polymer film.

In one aspect, the polymer film may be bonded to a cloth-like material without using additional adhesive lamination. The back sheet then comprises s the polymer film and the cloth-like material.

According to another aspect, there is provided a method of forming a back sheet of disposable hygiene product. The method includes positioning a back sheet station in a disposable hygiene product manufacturing line, the back sheet station having a fluid application device. The method also includes discharging, from the fluid application device, a sheet of material onto a laminate formed of an absorbent layer and a top sheet.

According to another aspect, there is provided a disposable hygiene product manufacturing line. The manufacturing line includes a back sheet station having a fluid application device configured to discharge a sheet of material on to a laminate, the sheet of material forming a back sheet and the fluid application device comprising a slot die extruder assembly.

Other objects, features, and advantages of the disclosure will be apparent from the following description, taken in conjunction with the accompanying sheets of drawings, wherein like numerals refer to like parts, elements, components, steps, and processes.

### BRIEF DESCRIPTION OF THE DRAWINGS

FIG. 1 is a block diagram showing a disposable hygiene product manufacturing line having in-line production of a back sheet, according to an embodiment;
FIG. 2 is a perspective view of a back sheet station positioned relative to the manufacturing line of FIG. 1, according to an embodiment;
FIG. 3 is an enlarged view of a portion of a fluid application device positioned relative to the manufacturing line of FIG. 1, according to an embodiment;
FIG. 4 is a perspective view of a fluid application device according to an embodiment;
FIG. 5 is a block diagram showing a method of forming a disposable hygiene product, according to one embodiment; and
FIG. 6 is a block diagram showing a method of forming a back sheet of disposable hygiene product, according to an embodiment.

### DETAILED DESCRIPTION

While the present disclosure is susceptible of embodiment in various forms, there is shown in the drawings and will hereinafter be described one or more embodiments with the understanding that the present disclosure is to be considered illustrative only and is not intended to limit the disclosure to any specific embodiment described or illustrated.

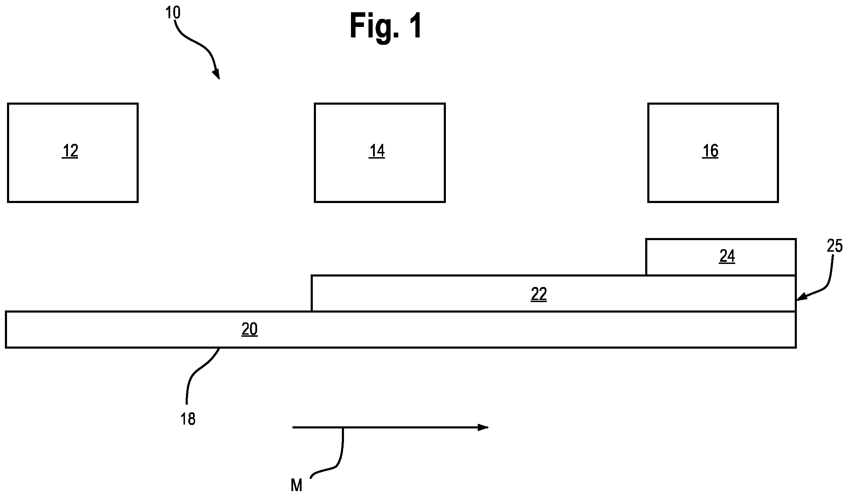

FIG. 1 is a block diagram of a disposable hygiene product manufacturing line 10 according to an embodiment described herein. In one embodiment, the disposable hygiene product manufacturing line 10 includes a back sheet station 12, a core layer station 14 and a top sheet station 16. A conveyor system 18 interconnects the back sheet station 12, the top core layer station 14 and the top sheet station 16. The conveyor system 18 may include a flexible member, such as a conveyor belt, or movable members, such as conveyor rollers to allow for transport or movement of material(s) between the different stations 12, 14, 16.

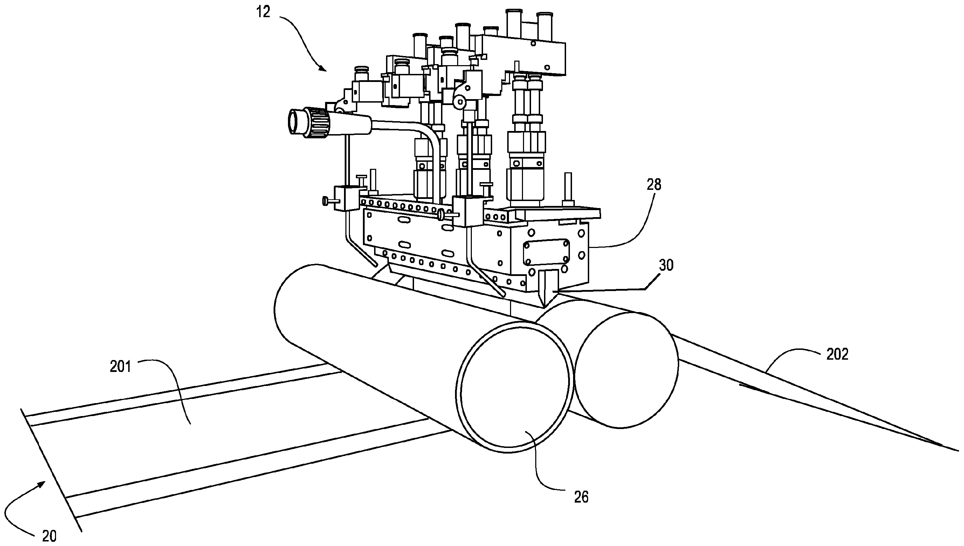

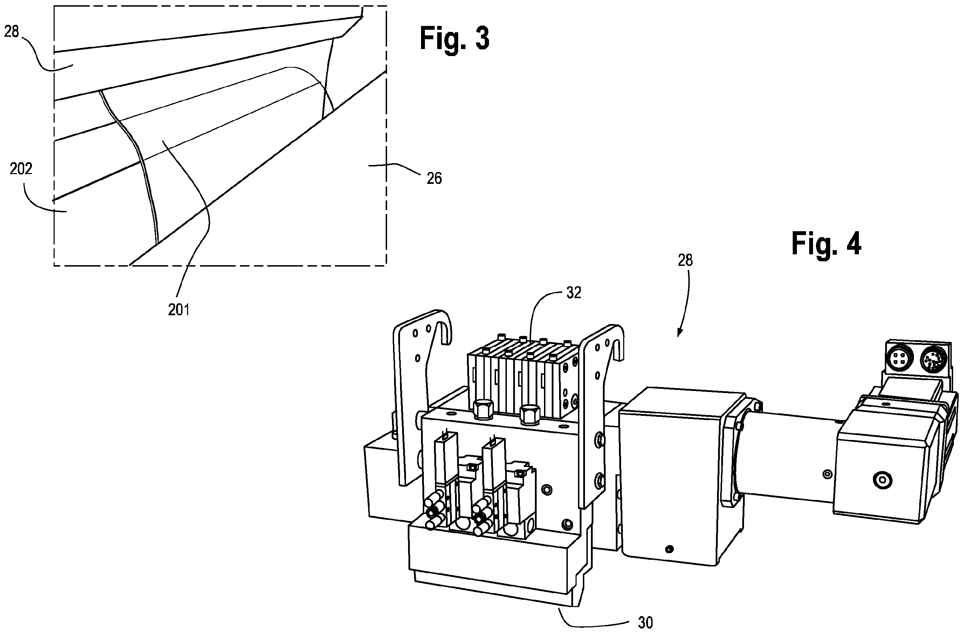

The back sheet station 12 is configured to produce a back sheet 20 in line with the manufacturing line 10. As will be detailed below, and shown in FIGS. 2 and 3, the back sheet station 12 is configured to produce a polymer film 201. The back sheet station 12 also includes s a roll of cloth-like material 202, such as a non-woven material. The polymer film 201 and cloth-like material 202 are laminated at the back sheet station 12 to form the back sheet 20. Thus, according to the invention, the back sheet 20 is, for example, a polymer film 201 alone, or a laminate of a polymer film 201 and an additional cloth-like material 202, such as a non-woven material.

The core layer station 14 is configured to produce an core layer 22, which may include, for example, an absorbent pad or layer, according to processes and techniques known to those having ordinary skill in the art. The core layer 22 may include absorbent synthetic or natural fibers or materials, including absorbent polymers with suitable absorption properties as understood by those having skill in the art. Alternatively, or in addition, the core layer station 14 is configured to receive a core layer 22 and, via the conveyor system 18, move the core layer 22 along the manufacturing line 10. The core layer station 14 is configured to apply the core layer 22 onto the back sheet 20 to form a back sheet 20 / core layer 22 laminate.

The top sheet station 16, according to one embodiment, is configured to unwind a roll of top sheet material to form a top sheet 24, in accordance with known procedures and techniques understood that by those having skill in art. The top sheet 24 may be applied onto the back sheet 20 / core layer 22 laminate and bonded thereto, for example, with an adhesive. In one embodiment, the top sheet material may be a permeable non-woven material. The back sheet 20, core layer 22 and top sheet 24 may be transported by the conveyor system 18.

The manufacturing line 10 may include one or more bonding stations (not shown) disposed between the back sheet station 12 and core layer station 14, and between the core layer station 14 and top sheet station 16. The bonding stations are configured to bond the back sheet 20, core layer 22 and top sheet 24 together to form a laminate. For example, a bonding station between the back sheet station 12 and core layer station 14 may be configured to apply an adhesive to the back sheet 20 so that the core layer 22 may be adhered to the back sheet 20 upon application thereon. A bonding station between the core layer station 14 and top sheet station 16 may include, for example, one more rollers to press the core layer 22 against the back sheet 20, and/or be configured to apply an adhesive on the core layer 22 so that the top sheet 24 may be adhered to the core layer 22. Another bonding station may be disposed downstream from the top sheet station 16 to, for example, press the top sheet 24 against the back sheet 20 / core layer 22 laminate to promote bonding thereto.

FIG. 2 is a perspective view of the back sheet station 12 positioned relative to manufacturing line 10, according to an embodiment. The back sheet station 16 may include one or more rollers 26 and a fluid application device 28 dispensing a material to form the polymer film 201. FIG. 3 is an enlarged view of a portion of the fluid application device 28 positioned relative to the manufacturing line 10, according to one embodiment. FIG. 4 is a perspective view of the fluid application device 28, according to one embodiment. Examples of fluid application devices 28 which may be used in the systems and method described herein include those shown and described in U.S. Pat. No. 8,413,848 to McGuffey and U.S. Pat. App. Pub. No. 2016/0303597 to McGuffey.

Referring to FIGS. 2 and 3, the one or more rollers 26 may be disposed at the back sheet station 12 for handling of the cloth-like material 202, such as the non-woven material, and the polymer film 201. As best shown in FIG. 2, the rollers 26 may bring the polymer film 201 and the cloth-like material 202 into pressed contact to form a laminate which may be used as the back sheet 20. It is understood, however, that the rollers 26 are optional. For example, in embodiments where the back sheet 20 is formed by the polymer film 201 alone, the rollers 26 maybe omitted.

With reference to FIGS. 2-4, the fluid application device 28 includes a slot die extruder assembly 30. In general, the slot die extruder assembly 30 includes a die adapter configured to receive a fluid material, a die plate, and one or more shims disposed between the die adapter and die plate. The die plate, die adapter and one or more shims are secured together in a fluidically sealed manner so as to prevent or limit unintended flow of the fluid material from the assembly 30. Internal channels (not shown) are configured to extrude the fluid material width-wise, i.e., in a direction transverse to a machine direction M.

The assembly 30 includes a discharge slot through which the fluid material may be discharged. The material may be discharged generally in the form of a curtain from the discharge slot, wherein the curtain forms the polymer film 201 used in the back sheet 20. In one embodiment, a width of the discharge slot generally corresponds to a desired width of the polymer film 201. However, the present disclosure is not limited to such a configuration. For example, in some embodiments, a width of the fluid material discharged from the slot forming polymer film 201 may be less than the width of the slot.

A material supply (not shown) may be positioned remotely of the manufacturing line 10 or may be included as part of the manufacturing line 10. The material supply is configured to deliver the material to the fluid application device 28. In one embodiment, one or more pump assemblies 32 may be directly mounted on the fluid application device 28, and in an embodiment, may be removably mounted. The pump assemblies 32 may be, for example, gear pumps or piston pumps. In an embodiment, the pump assembly 32 is a gear pump assembly comprising a plurality of interchangeable gear plates which may operate at different ratios as desired. Accordingly, a volume of material may be metered by the one or more pump assemblies 32 to the slot die assembly 30. Examples of such pump assemblies are shown and described in U.S. Pat. No. 8,413,848 and U.S. Pat. App. Pub. No. 2016/0303597, and in U.S. Pat. No. 6,688,498 to McGuffey, commonly owned with the present application. The pump assemblies 32 may meter the fluid according to a desired flow of the fluid application device. Alternatively, or in addition, a pump or metering assembly may be positioned at the material supply and is configured to deliver the material from the supply to the fluid application device 28.

In one embodiment, the fluid material may be a hot melt adhesive material. Further, in one embodiment, the hot melt adhesive material may be a polymer. Accordingly, the material, discharged from the discharge slot as a curtain of material, may form the sheet of polymer film 201. The polymer film 201 may be adhered to the cloth-like material 202 by way of contact with the material 202, and may be further secured to the cloth-like material 202 through pressing by the one or more rollers 26. Thus, in one embodiment, the back sheet 20 may be produced by dispensing a curtain of fluid material from the fluid application device 28 to form the polymer film 201 and in some embodiments, laminating the polymer film 201 to a cloth-like material 202 by contacting the polymer film 201 to the cloth-like material 202 and optionally pressing the film 201 and material 202 together with the rollers 26.

In some embodiments, it is envisioned that a step of laminating the polymer film 201 to the cloth-like material 202 may be carried out without the use of additional laminating steps or processes, such as the application of additional adhesives, heating or ultrasonic welding. In such embodiments, the polymer film 201 is discharged from the fluid application device 28 and contacted with the cloth-like material 202 at a sufficiently high temperature to allow for bonding. In other embodiments, however, additional laminating steps may be included. For example, additional heat may be applied to the polymer film 201 to maintain the film 201 at a suitable temperature for bonding to the cloth-like material 202. However, such a step, in contrast to conventional systems, does not require additional adhesives to bond the layers together.

Accordingly, in the embodiments described herein, the back sheet 20 may be fabricated at the manufacturing line 10, eliminating the need to transport rolls of previously produced polymer sheet material to the manufacturing site. In addition, equipment and process steps typically used for laminating a prefabricated polymer layer to a nonwoven layer, such as ultrasonic welding, heating or adhering may be omitted in the embodiments of the present application. As such, less equipment, space and manufacturing steps may be required. Further still, the pump assemblies 32 described herein are suitable for use with conventional disposable hygiene product manufacturing lines in that they require little operating space. As such, the gear or piston pumps 32 described herein may be more suitable than, for example, a system using planetary pumps.

The disposable hygiene product manufacturing line 10 described herein is suitable for producing disposable hygiene products such as adult diapers, baby diapers, feminine hygiene products or medical products using a solid film material. In one embodiment, the polymer film could range from 2 GSM to 24 GSM for such applications.

In the embodiments above, the fluid application device 28 may be a fully metered applicator having one or more pump assemblies 32 mounted directly to the applicator. The fluid application device 28 may also include a slot die assembly 30, such as an extruder nozzle assembly, configured to provide of a curtain of film material that could then be applied to a calendaring or gauging assembly to control a thickness or weight of the film.

Alternatively, the fluid application device 28 may be a pressure feed control applicator with a feed pump mounted on a remote metering system or material storage or tank system. The material storage or tank system may be a hopper fed unit or an enclosed extruder system, for example. The feed pumps may be metering gear pumps or screw extruder pumps.

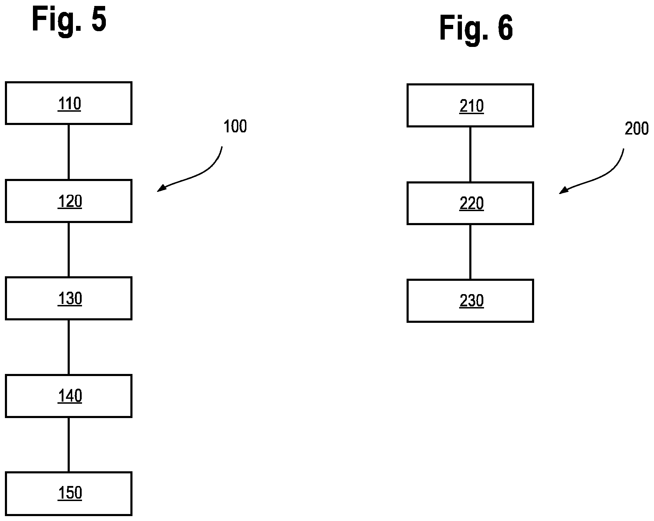

FIG. 5 is a diagram showing a method 100 of forming a disposable hygiene product, according to one embodiment. The method includes providing 110 a manufacturing line 10 having a back sheet station 12, a core layer station 14 and a top sheet station 16 connected by a conveyor system 18 and providing 120 a back sheet 20 at the back sheet station 12 and conveying the back sheet 20 in the machine direction 'M' along the conveyor system 18. The back sheet 20 may be provided by discharging a curtain of fluid material to form a polymer film 201 from a fluid application device 28. According to the invention, the back sheet 20 is provided by bonding the polymer film 201 to a cloth-like material 202, such as a non-woven material.

The method 100 may further include providing 130 a core layer 22 at the core layer station 14 and applying the core layer 22 onto the back sheet 20. In one embodiment, the core layer 22 maybe produced at the core layer station 14. In another embodiment, the core layer 22 may be separately fabricated and provided to the core layer station 14 for application to the back sheet 20.

The method 100 may further include providing 140 a top sheet 24 at the top sheet station 16. The top sheet 24 may be formed from a roll of material, such as a non-woven material. The method 100 also includes applying 150 the top sheet 24 onto the back sheet 20 and core layer 22. The method may further include, for example, bonding the core layer 22 to the back sheet 20 by adhering, heating or ultrasonic welding, and bonding the top sheet 24 to the back sheet 20 / core layer 22 laminate by adhering, heating, or ultrasonic welding. It is understood that other suitable bonding processes may be used as well.

FIG. 6 is a diagram showing a method 200 of forming a back sheet of disposable hygiene product, according to an embodiment. The method includes positioning 210 a back sheet station 12 in a disposable hygiene product manufacturing line 10, the back sheet station 12 including a fluid application device 28. The method further includes discharging 220, from the fluid application device 28, a curtain of material to form a polymer film 201. According to the invention, the method 200 further includes s bonding 230 the polymer film 201 to a cloth-like material 202, such as a nonwoven material, to form the back sheet 20. The bonding 230 may including pressing the polymer film 201 against the cloth-like material 202 with one or more rollers 26.

The disposable hygiene product may be formed by applying the core layer 22 and top sheet 24 to the back sheet 20 in the manner described above to form a product laminate 25. It is understood that additional hygiene product components not specifically described herein may be applied as well, including, but not limited to waist, leg and/or cuff elastics, tabs, fasteners and other suitable components. The product laminate may then be cut to size, for example, along its length, into individual disposable hygiene products.

In the present disclosure, the words "a" or "an" are to be taken to include both the singular and the plural. Conversely, any reference to plural items shall, where appropriate, include the singular.

## Claims

1. A method of forming a disposable hygiene product comprising:
providing a manufacturing line (10) having a back sheet station (12), a core layer station (14) and a top sheet station (16) connected by a conveying system (18);
providing a back sheet (20) at the back sheet station (12);
providing a core layer (22) at the core layer station (14);
applying the core layer (22) on the back sheet (20);
providing a top sheet (24) at the top sheet station (16); and
applying the top sheet (24) on the back sheet (20) and core layer (22);
wherein the back sheet station (16) comprises a fluid application device (28) configured to discharge a curtain of material to form a polymer film (201), wherein the back sheet (20) comprises the polymer film (201),
**characterised in that** the method further comprises providing a cloth-like material (202) at the back sheet station (12) and bonding the cloth-like material (202) to the polymer film (201) to form the back sheet (20).

2. The method of claim 1, wherein the fluid application device (28) comprises a slot die extruder assembly (30) configured to receive the material from a supply source and discharge the material from a discharge slot.

3. The method of claim 2, wherein the fluid application device (28) comprises a pump assembly (32) configured to supply the fluid to the slot die extruder assembly (30).

4. The method of claim 3, wherein the pump assembly (32) includes a gear pump.

5. The method of claim 3, wherein the pump assembly (32) includes a piston pump.

## Patentansprüche

1. Verfahren zur Herstellung eines Wegwerfhygieneprodukts, umfassend:
Bereitstellen einer Fertigungsstraße (10) mit einer Rückseitenlagenstation (12), einer Kernschichtstation (14) und einer Decklagenstation (16), die durch ein Fördersystem (18) verbunden sind;
Bereitstellen einer Rückseitenlage (20) an die Rückseitenlagenstation (12);
Bereitstellen einer Kernschicht (22) an die Kernschichtstation (14);
Aufbringen der Kernschicht (22) auf die Rückseitenlage (20) ;
Bereitstellen einer Decklage (24) an die Decklagenstation (16); und
Aufbringen der Decklage (24) auf die Rückseitenlage (20) und die Kernschicht (22);
wobei die Rückseitenlagenstation (16) eine Fluidauftragungsvorrichtung (28) umfasst, die ausgestaltet ist, um einen Materialvorhang auszutragen,
um einen Polymerfilm (201) zu bilden, wobei die Rückseitenlage (20) den Polymerfilm (201) umfasst,
**dadurch gekennzeichnet, dass** das Verfahren des Weiteren Bereitstellen eines tuchartigen Materials (202) an die Rückseitenlagenstation (12) und Bonden des tuchartigen Materials (202) an den Polymerfilm (201) umfasst, um die Rückseitenlage (20) zu bilden.

2. Verfahren nach Anspruch 1, wobei die Fluidauftragungsvorrichtung (28) eine Schlitzdüsenextruderanordnung (30) umfasst, die ausgestaltet ist, um das Material von einer Versorgungsquelle zu empfangen und das Material aus einem Austragungsschlitz auszutragen.

3. Verfahren nach Anspruch 2, wobei die Fluidauftragungsvorrichtung (28) eine Pumpenanordnung (32) umfasst, die ausgestaltet ist, um die Schlitzdüsenextruderanordnung (30) mit dem Fluid zu versorgen.

4. Verfahren nach Anspruch 3, wobei die Pumpenanordnung (32) eine Zahnradpumpe einschließt.

5. Verfahren nach Anspruch 3, wobei die Pumpenanordnung (32) eine Kolbenpumpe einschließt.

## Revendications

1. Procédé de formation d'un produit hygiénique jetable comprenant :
la fourniture d'une ligne de fabrication (10) comportant une station de feuille arrière (12), une station de couche centrale (14) et une station de feuille supérieure (16) reliées par un système de transport (18) ;
la fourniture d'une feuille arrière (20) au niveau de la station de feuille arrière (12) ;
la fourniture d'une couche centrale (22) au niveau de la station de couche centrale (14) ;
l'application de la couche centrale (22) sur la feuille arrière (20) ;
la fourniture d'une feuille supérieure (24) au niveau de la station de feuille supérieure (16) ; et
l'application de la feuille supérieure (24) sur la feuille arrière (20) et la couche centrale (22) ;
dans lequel la station de feuille arrière (16) comprend un dispositif d'application de fluide (28) configuré pour déverser un rideau de matériau pour former un film polymère (201), dans lequel la feuille arrière (20) comprend le film polymère (201),
**caractérisé en ce que** le procédé comprend en outre la fourniture d'un matériau de type tissu (202) au niveau de la station de feuille arrière (12) et la liaison du matériau de type tissu (202) au film polymère (201) pour former la feuille arrière (20).

2. Procédé selon la revendication 1, dans lequel le dispositif d'application de fluide (28) comprend un ensemble extrudeuse à matrice fendue (30) configuré pour recevoir le matériau en provenance d'une source d'alimentation et pour distribuer le matériau depuis une fente de distribution.

3. Procédé selon la revendication 2, dans lequel le dispositif d'application de fluide (28) comprend un ensemble pompe (32) configuré pour alimenter en fluide l'ensemble extrudeuse à matrice fendue (30).

4. Procédé selon la revendication 3, dans lequel l'ensemble pompe (32) comprend une pompe à engrenage.

5. Procédé selon la revendication 3, dans lequel l'ensemble pompe (32) comprend une pompe à piston.
